# EUROPEAN PATENT APPLICATION

(11) **EP 3 644 273 A1**
(43) Date of publication of application: **29.04.2020**
(21) Application number: 18202250.9
(22) Date of filing: 24.10.2018
(51) Int. Cl.: G06T 7/00

(54) **SYSTEM FOR DETERMINING IMAGE QUALITY PARAMETERS FOR MEDICAL IMAGES**

(71) Applicant: Koninklijke Philips N.V., 5656 AG Eindhoven (NL)
(72) Inventor: Buelow, Thomas, 5656 AE Eindhoven (NL); Young, Stewart, 5656 AE Eindhoven (NL); Bystrov, Daniel, 5656 AE Eindhoven (NL); von Berg, Jens, 5656 AE Eindhoven (NL); Wieberneit, Nataly, 5656 AE Eindhoven (NL); Harder, Tim Philipp, 5656 AE Eindhoven (NL); Nordhoff, Tanja, 5656 AE Eindhoven (NL)
(74) Representative: de Haan, Poul Erik

(57) **Abstract**

Disclosed is a system for analysis of medical images and which includes a data processing system, which is configured to determine quality parameter values for an image acquired using an imaging unit and to perform at least one of (a) and (b): (a) Reading one or more imaging parameter values, which are indicative of one or more properties of the imaging unit, one or more properties of an operator, who operates the imaging unit, and/or one or more properties of a subject from which the image is acquired; and determining a graphical representation depending on one or more quality parameter values of the image and one or more of the imaging parameter values, (b) Receiving user input which is indicative of a level of acceptance of the image and determining a graphical representation, depending on one or more of the quality parameter values and the user input.

## Description

### FIELD OF THE INVENTION

The present invention relates to a system, method, program element and computer readable medium for determining and evaluating quality image parameter values for assessing the quality of medical images.

### BACKGROUND OF THE INVENTION

Chest radiography is the most frequently performed of all medical imaging applications with 150 million exams per year in the United States. In radiologic departments, the functions of generating a radiograph and assessing its diagnostic quality are divided between the radiographer, also denoted as radiologic technologist, who operates the image acquisition unit on the one hand and the radiologist on the other hand. If an image shows severe quality degradation, for example caused by errors in positioning the patient relative to the imaging unit, the radiologist, when performing reject analysis, rejects the image classifying it as not being diagnostic. In such cases, it is necessary to acquire a repeat image from the patient. Repeat images, however, lead to additional costs, less productivity in the radiology department and higher radiation doses given to the patient. They also do not fit into a clinical workflow, which has to manage an ever-increasing amount of diagnostic data.

A similar problem exists in that some radiographers tend to directly reject images without consulting the radiologist in an effort to anticipate a possible rejection by the radiologist. This, however, leads to unnecessary repeat images, since the image was rejected by somebody who only has a basic medical training and is therefore not qualified to make a determination on whether or not the image is suitable for diagnosis.

It is known that improving the skills of radiographers to reduce the occurrence of repeat images requires effective communication between radiologists and radiographers which cannot be always guaranteed in the clinical environment. However, radiographers often receive only sparse and subjective feedback about the quality of their images. Therefore, radiographers often are not aware of the necessary improvements and how to achieve them.

Therefore, a need exists for a system, a method, a program element and a computer-readable medium, which allow a more efficient quality control for medical images.

This need is met by the subject-matter of the independent claims.

### SUMMARY OF THE INVENTION

Embodiments of the present disclosure pertain to a system for analysis of medical image data, which represent one or more two-dimensional or three-dimensional medical images, which are acquired from one or more human subjects using one or more imaging units. Each of the imaging units is configured to acquire images. The system comprises a data processing system which is configured to read and/or generate, for each of the medical images, one or more quality parameter values, which are indicative of a position parameter and/or an orientation parameter of one or more body portions of the subject relative to each other or relative to the respective imaging unit. The data processing system is configured for automatically or interactively performing at least one of (a) and (b). (a) Reading and/or generating, for each of one or more of the medical images, one or more imaging parameter values, which are indicative of one or more properties of the respective imaging unit, one or more properties of an operator, who operates the respective imaging unit, and/or one or more properties of a subject from which the image is acquired; and determining at least one graphical representation which is displayed by the data processing system and/or at least one analysis parameter value, each of which depending on one or more of the quality parameter values and one or more of the imaging parameter values. (b) Receiving and/or reading, for each of one or more of the medical images, user input which is indicative of a level of acceptance of the respective image, and determining at least one graphical representation which is displayed by the data processing system and/or at least one analysis parameter value, each of which depending on one or more of the quality parameter values and at least a portion of the user input.

The image data may be acquired using an X-ray imaging unit and/or using a magnetic resonance tomography imaging unit. The X-ray imaging unit may be configured for plain radiography and/or for X-ray computed tomography.

The data processing system may include a computer system having a processor and a memory for storing instructions processable by the processor. The processor may execute an operating system. The data analysis system may include a user interface. The user interface may be configured to allow a user to receive data from the data processing system and/or to provide data to the data processing system. The user interface may include a graphical user interface for displaying the graphical representations. The data processing system may include a display which provides the graphical user interface.

The body portions based on which the quality parameter values are generated may be imaged body portions so that the body portions are visible in the medical images. Examples for body portions are but are not limited to: a lung, a heart, a diaphragm, bones such as a clavicle, a rib bone, a vertebrae and a scapula.

The three-dimensional medical images may represent tomographic images. The tomographic images may be generated from two-dimensional images using a reconstruction algorithm. The two-dimensional medical images may be generated using planar radiography. By way of example, the medical images may be chest radiographs.

The data processing system may be configured to generate the quality parameter values depending on the medical image data. The quality parameter values may be determined automatically and/or interactively (i.e. requiring user intervention). The data processing system may implement a quality parameter calculation algorithm. The quality parameter calculation algorithm may include a machine learning algorithm. The machine learning algorithm may be trainable in a supervised and/or unsupervised fashion.

The data processing system may be configured to generate the imaging parameter values depending on image header data, which are included in the image data. The image header data may be generated using the imaging unit. Additionally or alternatively, the data processing system may be configured to receive user input indicative of one or more of the imaging parameter values.

The one or more analysis parameter values, which are determined depending on one or more of the imaging parameter values may be parameter values of a relationship (in particular parameter values of a deterministic relationship and/or parameter values of a statistical relationship) between one or more of the imaging parameter values on the one hand and one or more of the quality parameters values on the other hand. Examples for parameters of a statistical relationship are parameters of a regression model, such as a linear regression model.

The one or more analysis parameter values which are determined depending on the user input may be parameter values of a relationship (in particular parameter values of a statistical relationship) between the user input on the one hand and one or more of the quality parameters values on the other hand.

The level of acceptance may classify the image into one or more pre-defined classes of acceptance. The level of acceptance, which is input by the user, may be one of a plurality of levels which are selectable by the user using the user interface. The selectable levels may include a level which indicates that the image is unsuitable for diagnosis, a level which indicates that the image is suitable for diagnosis and is acceptable in view of one or more quality requirements and/or a level, which indicates that the image is suitable for diagnosis but is not acceptable in view of the one or more quality requirements. s

The level of acceptance may relate to quality requirements for one or more or all of the quality parameters. The user input may include input for a plurality of acceptances, each of which relating to one or more of the quality parameters so that the acceptances relate to different quality parameters.

According to a further embodiment, the data processing system is configured so that for each of the medical images, the one or more quality parameters include one or a combination of (i) to (iii). (i) One or more parameters of a position of a lung field within a two-dimensional field of view of the imaging unit and/or one or more parameters of an extent of the lung field relative to the field of view. (ii) One or more parameters of an orientation of at least one of the body portions relative to the imaging system. (iii) One or more parameters of an inhalation state of the subject during acquisition of the respective image. Specifically, the one or more parameters of the inhalation state may be indicative of whether or not the subject is in a maximum inhalation state or substantially in a maximum inhalation state.

The determination of one or more of the quality parameter values may include segmenting a lung field from the image. The segmentation may be performed using a machine learning algorithm. The machine learning algorithm may be trainable in a supervised and/or in an unsupervised fashion. Additionally or alternatively, the lung field may be segmented using atlas based registration and/or Procrustes analysis.

Additionally or alternatively, the determination of one or more of the quality parameter values may include determining a rectangle which surrounds the lung field. Each of the edges of the rectangle may be parallel to an edge of the image. The rectangle may have the smallest area of all rectangles, which surround the lung field. For one or more of the edges of the rectangle, the quality parameters may include a distance between the rectangle edge and a corresponding edge of the image. If the edge of the rectangle is located completely outside of the image, the distance may have a reversed sign compared to when the edge of the rectangle is at least partially located within the image.

The quality parameters may include a rotation angle of a rotation about a rotation axis. The rotation axis may be parallel or substantially parallel to a radiation-sensitive surface of the detector. By way of example, the rotation axis may correspond or substantially correspond to a longitudinal axis of the subject. The rotation angle may be determined using one or more pre-defined anatomical landmarks. The anatomical landmarks may be located at different distances relative to a radiation-sensitive surface of the detector. The landmarks may be located at a portion, in particular at a medial tip, of one or both clavicles, at a centerline of the spinal cord, at a medial line of the lung and/or at a spinous process of a vertebrae. The anatomical landmarks may be selected so that a distance between positions of the landmarks in the image depends on the rotation angle. The medial line may be determined using a registration of detected landmarks, which are located at a boundary of the lung field, wherein the registration is based on a statistical atlas of these landmarks. The statistical atlas may be generated using training data. The medial line once annotated in the statistical atlas may be projected into the image.

According to a further embodiment, the data processing system is configured to identify one or more of the medical images depending on the quality parameter values. By way of example, the identified images may represent images which do not have at least a pre-defined level of image quality. The identification of the images may be performed depending on quality requirements for one or more of the quality parameters. The quality requirements may be represented by thresholds and/or ranges for the quality parameter values.

According to a further embodiment, the data processing system is configured to display, for one or more of the medical images, a graphical representation, which is indicative of a parameter value of a corrective movement of the subject from which the image has been acquired. The corrective movement parameter value may be determined by the data processing system automatically or semi-automatically (i.e. requiring user intervention). The corrective movement parameter value may be determined by the data processing system so that through the corrective movement, one or more of the quality parameter values meet the quality requirements.

Additionally or alternatively, the graphical representation is indicative of a parameter value of an adjustment of the imaging unit. The adjustment parameter value may be determined by the data processing so that through the adjustment, one or more of the quality parameter values meet the image quality requirements. The adjustment parameter may relate to an adjustment of a collimator of the radiation source, which collimates X-rays which are generated by the radiation source.

According to a further embodiment, the graphical representation includes an animation sequence for visualizing the corrective movement parameter value and/or the adjustment parameter value. Thereby, the parameter value may be displayed in an animated fashion.

According to a further embodiment, for each of a plurality of the quality parameters, the user input is indicative of a level of acceptance for the respective quality parameter. Thereby, the user input is indicative of a plurality of levels of acceptance.

According to an embodiment, the graphical representation and/or the at least one analysis parameter values each of which being determined depending on the one or more of the quality parameter values and the one or more of the imaging parameter values, are indicative of one or more parameters of a relationship between the one or more of the imaging parameter values on the one hand and the one or more of the quality parameter values on the other hand.

According to a further embodiment, the graphical representation and/or the at least one analysis parameter values, each of which being determined depending on one or more of the quality parameter values and at least a portion of the user input are indicative of one or more parameters of a relationship between the portion of the user input on the one hand and one or more of the quality parameter values on the other hand.

The parameter of the relationship may be a parameter of a statistical relationship, such as a parameter of a statistical model. By way of example, the statistical model may be a linear regression model.

According to a further embodiment, the data processing system is configured to determine an acceptance level for a further medical image depending on the determined one or more analysis parameter values, in particular depending on the one or more parameter values of the relationship. The acceptance level may be determined automatically or semi-automatically (i.e. requiring user intervention).

According to a further embodiment, the determined one or more analysis parameter values are operation parameter values of a machine learning algorithm for automatically determining an acceptance level for a further medical image depending on one or more quality parameter values of the further image. In other words, an operation of the machine learning algorithm depends on the analysis parameter value.

According to a further embodiment, the data processing system is configured to use an image analysis algorithm, which is used for determining one or more of the quality parameter values using at least a portion of the medical image data. The image analysis algorithm may be based on machine learning. The machine learning may be performed in a supervised and/or unsupervised fashion.

Embodiments of the present invention pertain to a method for analyzing medical image data, wherein the medical image data represent one or more two-dimensional or three-dimensional medical images which are acquired from one or more human subjects using one or more imaging units, wherein each of the imaging units is configured to acquire images. The method comprises reading and/or generating, using the data processing system, for each of the medical images, one or more quality parameter values, of a position parameter and/or an orientation parameter of one or more body portions of the subject relative to each other or relative to the respective imaging unit. The method further comprises one or a combination of (a) and (b): (a) Reading and/or generating, using the data processing system, for each of one or more of the medical images, one or more imaging parameter values, which are indicative of one or more properties of the respective imaging unit, one or more properties of an operator, who operates the respective imaging unit, and/or one or more properties of a subject from which the image is acquired; and determining, using the data processing system, at least one graphical representation which is displayed by the data processing system and/or at least one analysis parameter value, each of which depending on one or more of the quality parameter values and one or more of the imaging parameter values. (b) Receiving and/or reading, using the data processing system, for each of one or more of the medical images, user input which is indicative of a level of acceptance of the respective image, and determining, using the data processing system, at least one graphical representation which is displayed by the data processing system and/or at least one analysis parameter value, each of which depending on one or more of the quality parameter values and at least a portion of the user input.

Embodiments of the present disclosure pertain to a program element for analyzing medical image data, wherein the medical image data represent one or more two-dimensional or three-dimensional medical images which are acquired from one or more human subjects using one or more imaging units, wherein each of the imaging units is configured to acquire images. The program element, when being executed by a processor of the data processing system, is adapted to carry out: reading and/or generating, using the data processing system, for each of the medical images, one or more quality parameter values of a position parameter and/or an orientation parameter of one or more body portions of the subject relative to each other or relative to the respective imaging unit. The program element, when being executed by a processor of the data processing system, is adapted to carry out one or a combination of (a) and (b): (a) Reading and/or generating, using the data processing system, for each of one or more of the medical images, one or more imaging parameter values, which are indicative of one or more properties of the imaging unit, one or more properties of an operator, who operates the respective imaging unit, and/or one or more properties of a subject from which the image is acquired; and determining, using the data processing system, at least one graphical representation which is displayed by the data processing system and/or at least one analysis parameter value, each of which depending on one or more of the quality parameter values and one or more of the imaging parameter values. (b) Receiving and/or reading, using the data processing system, for each of one or more of the images, user input which is indicative of a level of acceptance of the respective image, and determining, using the data processing system, at least one graphical representation which is displayed by the data processing system and/or at least one analysis parameter value, each of which depending on one or more of the quality parameter values and at least a portion of the user input.

Embodiments of the present disclosure pertain to a computer readable medium having stored thereon the computer program element described herein.

### BRIEF DESCRIPTION OF THE DRAWINGS

Fig. 1 is as schematic illustration of a system for analysis of medical image data according to an exemplary embodiment;
Figs. 2A and 2B schematically illustrate determination of quality parameters of a position and quality parameters of an extent of a lung field relative to the field of view, wherein the parameters are determined by a data processing system of the exemplary system, which is shown in Fig. 1;
Figs. 3A and 3B schematically illustrate determination of a quality parameter value which is indicative of an inhalation state of a subject during acquisition of an image, wherein the determination is performed by the data processing system of the exemplary system, which is shown in Fig. 1;
Figs. 4A, 4B, 5A and 5B schematically illustrate determination of a quality parameter, which is an angle of rotation of the subject relative to the detector, wherein the determination is performed by the data processing system of the exemplary system, which is shown in Fig. 1;
Figs. 6A and 6B schematically illustrate graphical representations, which are displayed by the data processing system of the exemplary system, which is shown in Fig. 1 for indicating a corrective movement;
Figs. 7A to 7C schematically illustrate graphical representations, which are generated by the data processing system of the system which is shown in Fig. 1 using quality parameter values and imaging parameter values; and
Fig. 8 schematically illustrates a graphical user interface element for receiving user input, which is indicative of a level of acceptance of an image, wherein the graphical user interface element is displayed to the user by the data processing system of the exemplary system, which is shown in Fig. 1.

### DETAILED DESCRIPTION OF THE EMBODIMENTS

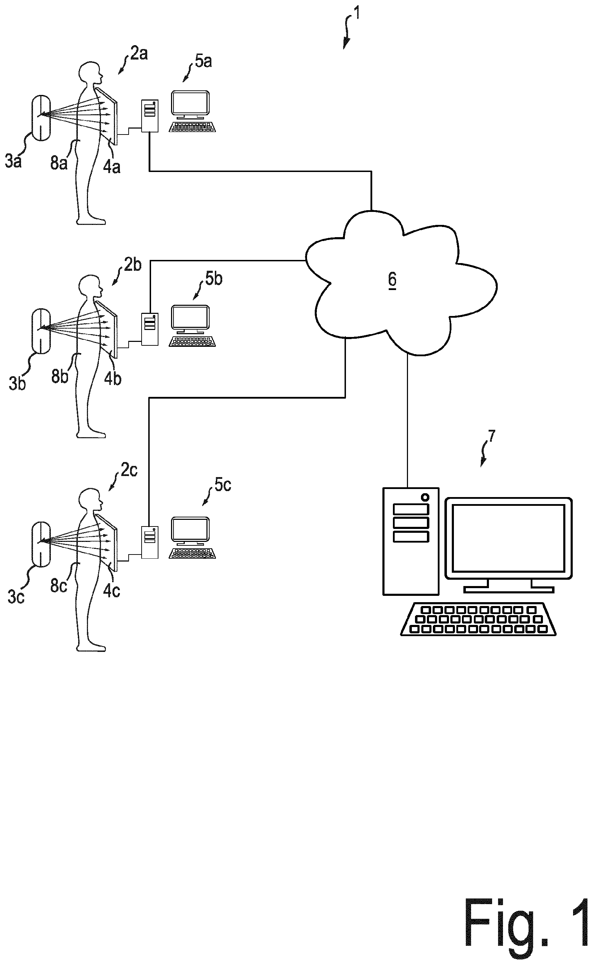

Fig. 1 is as schematic illustration of a system 1 for analysis of medical image data according to an exemplary embodiment. The system 1 includes a plurality of imaging units 2a to 2c, each which being configured to acquire X-ray images using planar projection radiography. However, it is to be understood that the present disclosure is not limited to systems for planar projection radiography, but can be used with any medical imaging unit, which is configured to generate medical image data, which represent one or more two-dimensional and/or three-dimensional medical images. Examples of such imaging units are, but are not limited to: X-ray computed tomography systems, magnetic resonance tomography systems, and scintigraphy systems.

In the exemplary embodiment which is shown in Fig. 1, each of the imaging units 2a to 2c includes a radiation source 3a to 3c and a radiation-sensitive detector 4a to 4c which is configured to detect X-ray imaging radiation, which is emitted from the radiation source 3a to 3c. Each of the imaging units 2a to 2c is configured to acquire projection X-ray images from a subject 8a to 8c, which is positioned between the radiation source 3a to 3c and the radiation-sensitive detector 4a to 4c. Each of the radiation sources 3a to 3c is configured to be substantially a point source for the imaging radiation. The imaging radiation traverses at least a portion of the body of the subject 8a to 8c before being incident on the radiation-sensitive detector 4a to 4c.

The system 1 includes, for each of the imaging units 2a to 2c, a computer system 5a to 5c, which is operated by a radiographer who positions the subject 8a to 8c between the X-ray-radiation source 3a to 3c and the detector 4a to 4c so that a body portion of the subject 8a to 8c is imaged using the X-rays which are emitted from the radiation source 3a to 3c. The imaged body portions of the subjects 8a to 8c may be, for example, the chests of the subjects so that the image data, which are generated by the imaging units 2a to 2c represent a chest radiographs.

Manual evaluation of the quality of the medical images usually suffers from subjective variation across multiple radiographers and radiologists so that some medical images are rejected by radiographers, which are found to be acceptable by the radiologists. This usually leads to a high number of repeat images, which, in turn, leads to additional costs, less productivity in the radiology department and higher radiation doses given to the patients. This also does not fit into a clinical workflow, which has to manage an ever-increasing amount of diagnostic data.

A similar problem exists in that, many radiographers are not sufficiently trained to acquire images in such a manner so that almost each image meets a desirable quality level. It is known that improving the skills of radiographers to reduce the occurrence of repeat images requires effective communication between radiologists on the one hand and radiographers on the other hand. It has, however, been shown that this cannot be always ensured in the clinical environment. Typically, radiographers often receive only sparse and subjective feedback about the quality of their images. Therefore, radiographers often are not aware of the required improvements and how they can be achieved.

However, the inventors have shown that a system and method for analysis of medical image data can be provided which allow efficient quality control for medical images.

Specifically, the system 1 is configured to automatically generate, for each of the medical images, one or more quality parameters values, which are indicative of a parameter of a position and/or of a parameter of an orientation of one or more body portions of the subject 8a relative to each other or relative to the imaging unit 2A. Examples of such quality parameter values are explained in the following. The quality parameter values for an image which has been acquired using one of the imaging systems are automatically generated by a data processing system. It has been shown by the inventors that automatic generation of the quality parameter values lead to objective, unbiased and reproducible results, which can be used to train the radiographers and which eliminate subjective variation in the quality assessment of the images.

The data processing system is a distributed computer system, which includes computer systems 5a to 5c, each of which being in signal communication with one of the imaging units 2a to 2c and being operated by one or more radiographer. The data processing system further comprises a central computer system 7 which may be, for example, operated by a radiologist. The central computer system 7 may be a dedicated server that receives all images acquired using the imaging units 2a to 2c and which are to be analyzed with regards to quality. The computer systems 5a to 5c and 7 are in signal communication with each other via a computer network 6, which may include a LAN (local area network) and/or the Internet.

The following includes a description of how quality parameter values are generated. The description relates to the operation of one or more of the computer systems 5a to 5c and 7 which analyze images which are acquired using one or the imaging units 2a to 2c.

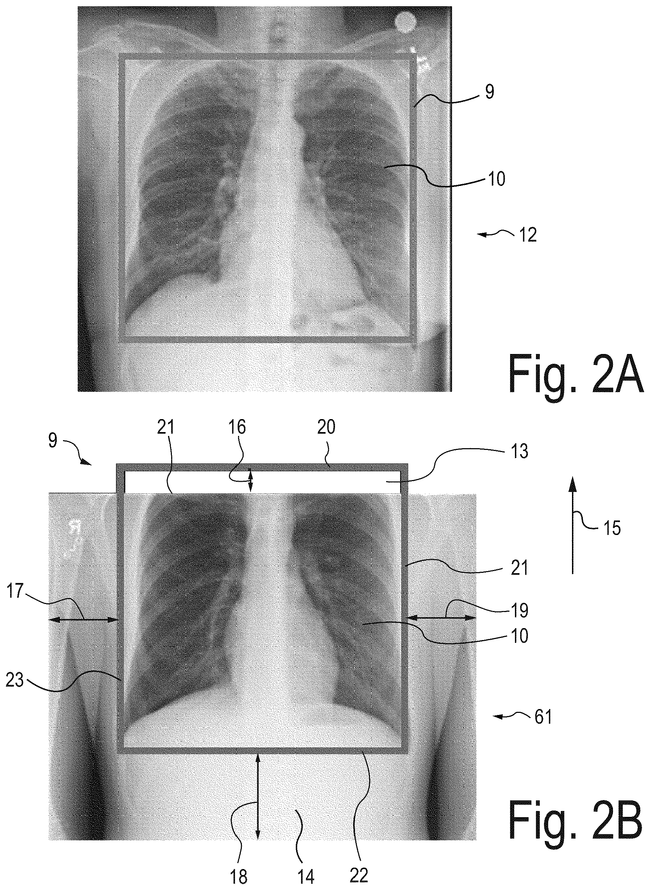

Figs. 2A and 2B schematically illustrate generation of quality parameter values of one or more parameters of a position of a lung field within the two-dimensional field of view of the imaging unit and of one or more parameters of an extent of the lung field relative to the extent of the field of view.

These quality parameters are configured to allow detection of under-collimation and over-collimation in the images. Over-collimated images show only a portion of the lung so that only a portion of the necessary information may be available to perform a diagnosis. On the other hand, under-collimated images show body portions which do not need to be imaged to perform the diagnosis so that these body portions are exposed to radiation, which can be avoided through proper collimation.

The position of the lung field relative to the field of view can be adjusted by adjusting a position of the collimator and/or by adjusting a relative position between the subject and the radiation-sensitive detector. The extent of the lung field relative to the field of view can be adjusted by adjusting an opening formed by the collimator.

In order to determine the quality parameter values, the data processing system may be configured to segment the lung field from the chest radiograph. Segmentation of the lung field may be performed using a machine learning algorithm. The machine learning algorithm may be configured to be trainable in a supervised and/or unsupervised fashion. The machine learning algorithm may include an artificial neural network. The machine learning algorithm may be configured to generate binary or probabilistic pixel classification data. The probabilistic pixel classification data may include, for each of a plurality of pixels of an output image of the machine learning algorithm, one or more values which indicate a probability that the respective pixel is part of the lung field.

As is illustrated in Figs. 2A and 2B, the data processing system determines, depending on one or more parameters values of an extent and a position of the lung field 10 (such as depending on the segmented lung field), a rectangle 9 which surrounds the lung field 10, wherein the edges of the rectangle 9 are parallel to the edges of the image 12. The rectangle 9 may represent the rectangle having the smallest area of all rectangles which surround the lung field.

As can be seen from Fig. 2A, the rectangle 9 is fully located within the field of view, thereby indicating that no over-collimation is present. This allows establishing a diagnosis based on each portion of the lung.

Further, as can be seen by comparing the rectangle 9 with the field of view, the extent of the lung field 10 represents a predominant portion of the field of view. Thereby, only a small portion of the body outside the lung field is exposed to the imaging radiation. Therefore, Fig. 2A shows an image in which the position and extent of the lung field relative to the field of view is suitable for diagnosis and which incurs only a small radiation dose to body portions outside the lung field.

On the other hand, as is illustrated in Fig. 2B, in a second image 61, the rectangle 9 is partially located outside of the field of view so that the image is over-collimated with respect to the top edge of the rectangle 9. Thereby, only a lower portion of the lung field 10 is captured in the image and an undesirable large portion 14 of the remaining body is exposed to X-ray radiation.

As is illustrated in Fig. 2B, the data processing system determines for each of the edges 20, 21, 22 and 23 of the rectangle 9, a distance 16, 17, 18 and 19 between the respective edge 20, 21, 22 and 23 and a corresponding edge of the image. For each of the edges 20, 21, 22 and 23 of the rectangle 9, the respective edge and its corresponding edge of the image 12 are parallel relative to each other and both represent a same circumferential side. In other words, both, the respective edge and its corresponding edge of the image represents a horizontal top edge, a horizontal bottom edge, a vertical left side edge, or a vertical right side edge.

If an edge of the rectangle 9 is completely located outside of the image (such as the top edge 20), the distance is measured in negative values. Therefore, a negative value indicates over-collimation with respect to the corresponding edge of the image. On the other hand, if the edge is at least partially located within the image (such as the edges 20, 21, 22 and 23), the distance is measured in positive values. If the positive value of a distance exceeds a pre-defined threshold, this indicates under-collimation with respect to the corresponding edge. The distances 16, 17, 18 and 19 therefore represent parameters of a position and an extent of the lung field relative to the field of view and are field of view quality parameters, which are used by the data processing system to perform an assessment of image quality.

The data processing system is configured to assess the field of view quality parameters using pre-defined threshold values and/or ranges, which are stored in a storage device of the data processing system. Thereby, immediately after the image 12 has been captured from the subject, the data processing system determines whether the field of view quality parameter values meet the quality requirements represented by the thresholds and/or ranges. This allows providing the radiographer with immediate feedback, whether or not the image meets the pre-defined quality requirements. Thereby the image quality is improved by establishing a feedback-loop.

The data processing system may store, in a storage device of the data processing system, for each of the edges 20, 21, 22 and 23 a pre-defined range for the respective distance. By way of example, one or more of the ranges may have a value of zero as its lower limit in order to prevent over-collimation. If each of the distances lies within its pre-defined range, the data processing system classifies the image as meeting the the field of view quality requirements. If at least one of the distances lies outside its pre-defined range, the data processing system classifies the image 12 as not meeting the field of view quality requirements.

The data processing system is configured to indicate to the radiographer, whether the image meets the field of view quality requirements in order to provide him with immediate feedback. Thereby, image quality can be improved by establishing a feedback-loop.

It is to be understood that the present invention is not limited to the determination of the distances 16, 17, 18 and 19, as described above in connection with Fig. 2B in order to assess over- and/or under-collimation. Additionally or alternatively, the data processing system may determine a ratio of an area portion 13 (shown in Fig. 2B) of the rectangle 9 (or the area portion of the lung field), which is located outside the field of view, to the total area of the rectangle 9. Additionally or alternatively, the data processing system may be configured to determine the ratio of the area represented by the rectangle 9 (or the area of the lung field) to the area of the field of view of the medical image 12, 61.

The data processing system is further configured to indicate to the radiographer, via the graphical user interface, how an improved image can be acquired through an adjustment of the subject and/or to the imaging unit. By way of example, as is illustrated in Fig. 2B, the graphical user interface presents to the user a graphical representation, such as an arrow 15 which indicates that, in order to obtain an image which fulfils the pre-defined field of view quality requirements, the X-ray collimator of the radiation source has to be adjusted by moving it upwards. Thereby, it is ensured that the radiographer acquires repeat images which meet the field of view quality requirements. Additionally or alternatively, the data processing system may be configured to display, via the graphical user interface, an amount of a necessary displacement of the collimator, expressed in units of length, which places the lung field in a correct position relative to the field of view.

As such, a radiographer, who is insufficiently trained directly benefits from the indication of the corrective movement because it is indicated to the radiographer how the quality of the images can be improved rather than merely presenting the results.

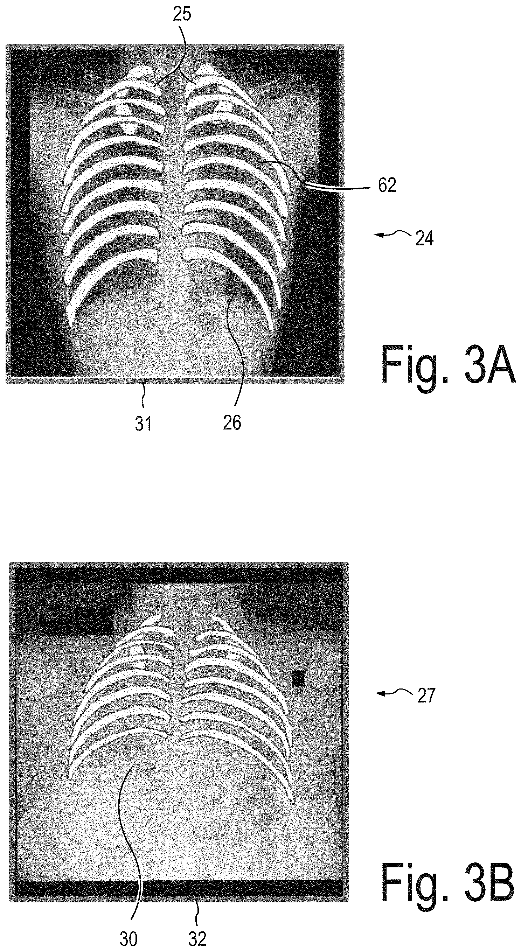

As is explained in the following with reference to Figs. 3A and 3B, the data processing system of the exemplary embodiment is further configured to determine, depending on the image data, one or more parameter values of an inhalation state of the subject during acquisition of the image. Images which represent a maximum inhalation state of the subject have been proven to ensure a reliable diagnosis so that radiologists are well experienced in the diagnosis based on such images. If the subject holds its breath during acquisition of the image, this also avoids blurring the image as a result of motion artifacts. Also, the maximum inhalation state reveals the full lung field in the image. If the chest is not imaged in the maximum inhalation state, the image may show opacities due to regions of denser tissue that could erroneously be mistaken as potentially pathologic consolidations.

In order to obtain one or more parameter values of the inhalation state of the subject, the data processing system determines the number of posterior ribs pairs, wherein each rib of the rib pair at least partially overlaps with the lung field. Additionally or alternatively, the data processing system may be configured to determine the number of posterior rib pairs, wherein each rib of the rib pair is located above the diaphragm. Additionally or alternatively, the data processing system may determine a parameter value, which is indicative of a vertical position of the diaphragm.

By way of example, the data processing system may be configured to determine centerlines of the posterior ribs of the lung field. The centerlines may be determined using a machine learning algorithm and/or using a registration of detected landmarks, wherein the registration is based on a statistical atlas of these landmarks. Based on the determined centerlines and using dynamic programming, pairs of lung fields may be identified. The detection algorithm may propagate upwards and downwards until either no more further candidate for a rib pair is detected or until a break signal occurs. An example of such a break signal may be the diaphragm, which stops the downward propagation of the detection algorithm.

Fig. 3A illustrates an image 24, of a subject which is in the maximum inhalation state. Depending on the image data of the image 24, the data processing system determined ten rib pairs, such as the rib pair 25, wherein each rib of the rib pairs at least partially overlaps with the lung field. Further, as can be seen from Fig. 3A, the diaphragm 26 is located comparatively close to the bottom edge of the image 24. On the other hand, Fig. 3B illustrates an image 27 which is not acquired at the maximum inhalation state. The data processing system only determined eight rib pairs, each of which being least partially overlapping with the lung field. Further, the diaphragm 30 is located at a comparatively distant position from the bottom edge 32 of the image 27.

Additionally or alternatively, the data processing system may be configured to detect the number pairs of anterior ribs (rather than posterior ribs), wherein each rib of the pairs at least partially overlaps with the lung field and/or is at least partially located above the diaphragm.

Depending on the determined one or more parameter values of the inhalation state, the data processing system checks, whether the image meets the inhalation state quality requirements. By way of example, the data processing system may use, as a threshold value, a pre-defined number of rib pairs, wherein each of the ribs is least partially overlapping with the lung field and/or is at least partially located above the diaphragm. The threshold value may relate to posterior rib pairs and/or to anterior rib pairs. By way of example, the threshold value may require at least 10 posterior rib pairs and/or at least 6 anterior rib pairs.

Based on the inhalation state quality parameter values, the data processing system determines that image 24 (shown in Fig. 3A) meets the inhalation state quality requirements and further determines that image 27 (shown in Fig. 3B) does not meet the inhalation state quality requirements so that a repeat image needs to be acquired. This result is indicated to the radiographer via the graphical user interface to provide him with immediate feedback.

The data processing system is further configured to determine a rotation angle of the subject's body about its longitudinal axis. The orientation angle may be determined depending on one or more anatomical landmarks which are identified in the image. The landmarks may be located in the subject's body at different distances from a radiation-sensitive surface of the detector. The distance may be measured in a direction, which is normal to at least a portion of the radiation-sensitive surface. Thereby, a displacement of the landmarks in the image is indicative of the rotation angle of the body if a typical distance between the landmarks, measured in the direction which is normal to at least the portion of the radiation-sensitive surface of the detector, is assumed. Additionally or alternatively, the term (dₗ-dᵣ)/(dₗ+dᵣ) may be used as a parameter of the orientation, wherein dₗ represents the distance (in particular the smallest distance) of the detected landmark of the left clavicle tip from the medial line of the lung and dᵣ represents the distance (in particular the smallest distance) of the detected landmark of the right clavicle tip from the medial line of the lung. The landmarks may be identified using a machine learning algorithm, which may be implemented using an artificial neural network.

Determining the rotation angle about the subject's longitudinal axis allows to check whether the frontal plane of the subject was oriented substantially parallel to the radiation-sensitive surface of the detector when the image was acquired. Such an orientation allows a more reliable diagnosis, since the image represents a symmetrical reproduction of the thorax.

By way of example, the data processing system may be configured to determine a location of the medial tips of both clavicles in the image as landmarks. Additionally or alternatively, landmarks which are used by the data processing system may include but are not limited to one or a combination of: a medial line of the lungs, a centerline of the spinal cord and/or one or more spinous processes. The spinous process is a bony outgrowth that projects backwardly and downwardly from the neural arch.

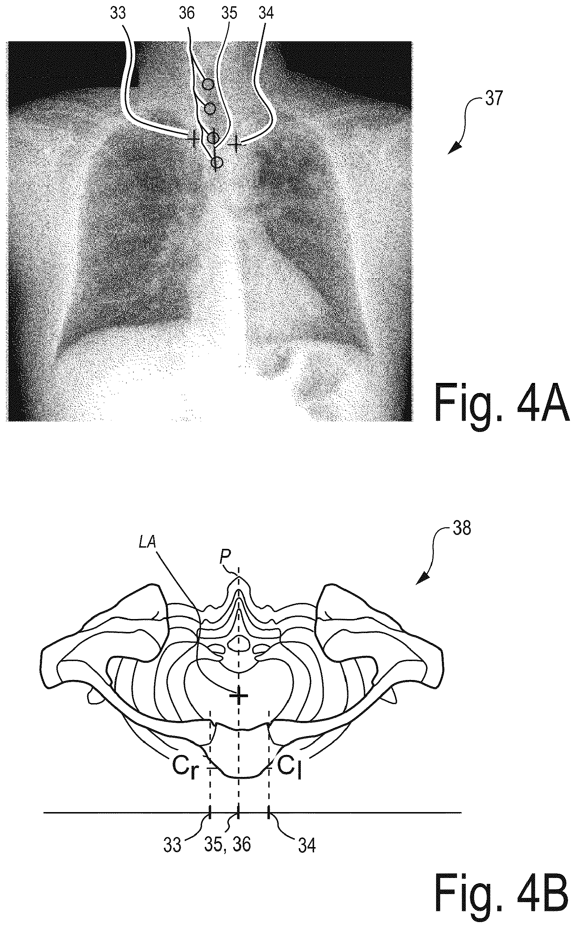

Fig. 4A schematically illustrates an image 37 and Fig. 4B schematically illustrates a corresponding cross-section 38 parallel to the transverse plane of the subject's body. As can be seen from Fig. 4A, the data processing system has determined for a plurality of vertebrae, locations 36 of the spinous processes. The spinous processes represent the most posterior part of the body, which is visible in the image. Furthermore, the data processing system has determined the locations 33 and 34 of the medial tips of both clavicles. The medial tips of the clavicles are the most anterior part of the body, which is visible in the image. Moreover, the data processing system has calculated a location 35 of a vertical medial line of the lung. The medial line of the lung may be determined as described above. Depending on the determined locations, the data processing system determines the rotation angle of the subject about its longitudinal axis. Since in the image 37 of Fig. 4A, the locations 36 of the spinous processes substantially lie on the medial line 35 and are located at substantially equal distances from the locations 33, 34 of the medial tips of the clavicles, the data processing system determines that the subject's frontal plane is substantially parallel to at least a portion of the light-sensitive surface of the detector, which, by definition, corresponds to a rotation angle of zero.

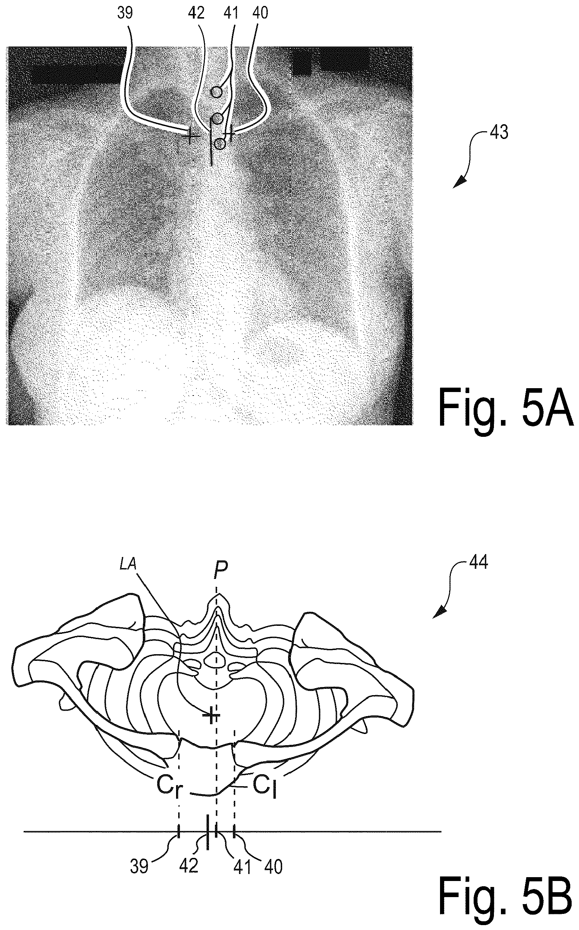

Fig. 5A schematically illustrates an image 43 and Fig. 5B a corresponding cross-section 44 parallel to the transverse plane of the subject's body. As can be seen from Fig. 5A, the data processing system has determined the locations 41 of a plurality of spinous processes. Furthermore, the data processing system has determined the locations 39 and 40 of the medial tips of both clavicles, as well as a location 42 of the vertical medial line of the lung. As can be seen from Fig. 5A, the locations 41 of the spinous processes are located distant from the location 42 of the medial line of the lung. Further, the locations 39 and 40 of the medial tips of the clavicles are asymmetric relative to the positions 41 of the spinous processes and the location of the medial line 42 of the lung. This indicates that the frontal plane of the subject's body is angled relative to at least a portion of the radiation-sensitive detector surface. Such a rotation can lead to artifacts in the image which in turn reduces the reliability of the diagnosis. For the image 43 which is shown in Fig. 5A, the data processing system has calculated a rotation angle of 5 degrees and therefore determined that the image does not meet the pre-defined rotation angle quality requirements.

By way of example, a pre-defined upper limit for the absolute value of the rotation angle may have a value which is less than 10 degrees, or less than 7 degrees, or less than 6 degrees. The value of the pre-defined upper limit may be greater than 2 degrees, greater than 3 degrees or greater than 4 degrees. If the rotation angle is outside the pre-determined range, the data processing system indicates to the radiographer that the image does not meet the rotation angle quality requirements.

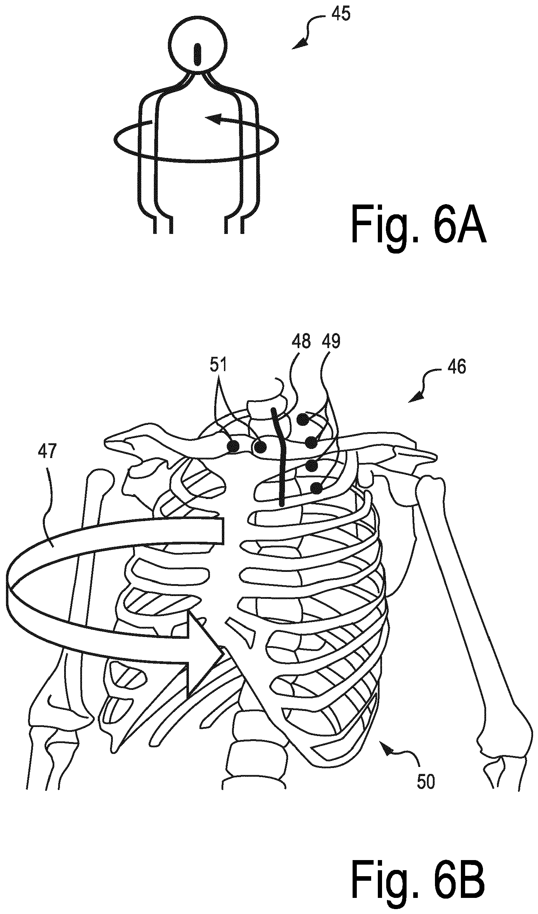

Furthermore, as is shown in Fig. 6A, the data processing system generates a graphical representation 45, which is an icon and which is indicative of a rotation direction of a corrective rotation of the subject about its longitudinal axis. The corrective rotation corrects the rotation angle of the body, leading to a rotation angle which is within the pre-determined range. Additionally or alternatively, the graphical user interface may indicate a rotation angle of the corrective rotation in degrees. This helps the radiographer to acquire a repeat image, which meets the rotation angle quality requirements and thereby avoids further positioning mistakes when the repeat image is acquired.

A further embodiment for a graphical representation 46 which is displayed by the data processing system is shown in Fig. 6B. The graphical representation 46 of Fig. 6B shows a three-dimensional model of the bone structure 50 of the subject's chest as well as an indicator 47, which indicates the direction of rotation to correct the subject's orientation so that the rotation angle meets the rotation angle quality requirements. The graphical representation may also show one or more landmarks in the three-dimensional model (i.e. the locations 51 of the medial tips of the clavicles, the locations 49 of the spinous processes and/or the medial line 48 of the lung). The data processing system may be configured so that the graphical representation 46, which is shown in Fig. 6B, is only presented on request of the user and/or in one or more pre-defined operation modes. By way of example, such pre-defined operation modes are provided for training purposes.

It is conceivable that the graphical representations of Figs. 6A and 6B are configured as an animated sequence of images so as to visualize the corrective movement in an animated manner. This makes it even more easier for the radiographer to see which corrective movement is necessary in order to acquire an image which meets the rotation angle quality requirements. Therefore, such a representation even more effectively avoids mistakes in positioning the subject when acquiring the repeat image.

The graphical representations of Figs. 6A and 6B provide immediate feedback to the radiographer who acquired the image and thereby improve image quality by establishing a feedback-loop.

As is shown in Fig. 1, the data processing system is configured as a distributed system which includes, for each of the imaging units 2a to 2c, a computer system 5a, 5b and 5c which receives image data from the respective imaging unit 2a to 2c and determines one or more quality parameter values as has been discussed with reference to Figs. 2A to 5B.

The data processing system comprises a further computer system 7, which is in signal communication with the computer systems 5a to 5c via a computer network 6 and which is configured to evaluate the quality parameter values which are calculated in each of the computer systems 5a to 5c. The computer system 7 further receives from each of the computer systems 5a to 5c one or more imaging parameter values, which are indicative of one or more properties of the imaging unit, one or more properties of an operator, who operates the respective imaging unit, and/or one or more properties of a subject from which the image is acquired. However, it is conceivable that the functionality of the computer system 7 is, additionally or alternatively, provided by one or more of the computer systems 5a to 5c.

Examples for properties of the imaging unit are but are not limited to: a device identifier (such as a serial number generated upon assembly of the imaging unit), a parameter of an operational state of the imaging unit (such as an energy of the X-rays used for acquiring the image, a radiation dose to which the subject is exposed, adjustment parameters of a collimator of the imaging unit and/or parameters of a position and an orientation of the radiation sensitive detector of the imaging unit, and the duration of the exposure for acquiring the image), and technical equipment of the imaging unit.

Examples of properties of the subject, from which the image is acquired are but are not limited to: the age and/or sex of the subject, body parameters such as the body height and/or the body mass index (BMI), diagnostic data acquired from the X-ray image or from other medical investigations, and reasons for acquiring the image. Examples of properties of the operator who operates the respective imaging unit are but are not limited to an operator identifier for uniquely identifying the operator and a parameter value, which is indicative of a parameter of professional experience.

Depending on one or more quality parameter values and one or more imaging parameter values, the computer system 7 determines an analysis parameter value. The determination of the analysis parameter value may be performed automatically or semi-automatically (i.e. requiring user intervention). Thereby, it is possible to perform an analysis for identifying the causes why images do not meet the quality requirements. In particular, the determination of the analysis parameter value may include a root-cause analysis. The root-cause analysis may determine the root cause why not all images (or less images than a pre-defined threshold) meet the quality requirements, which may be defined by thresholds and/or ranges for the quality parameters. Additionally or alternatively, the determination of the analysis parameter value may include performing a statistical analysis for determining a statistical relationship between one or more of the quality parameter values on the one hand and one or more of the imaging parameter values on the other hand.

The data processing system is further configured to generate a graphical representation which is displayed by the graphical user interface of the data processing system and which is determined using one or more quality parameter values and one or more imaging parameter values. The graphical representation may be generated automatically or semi-automatically (i.e. requiring user intervention).

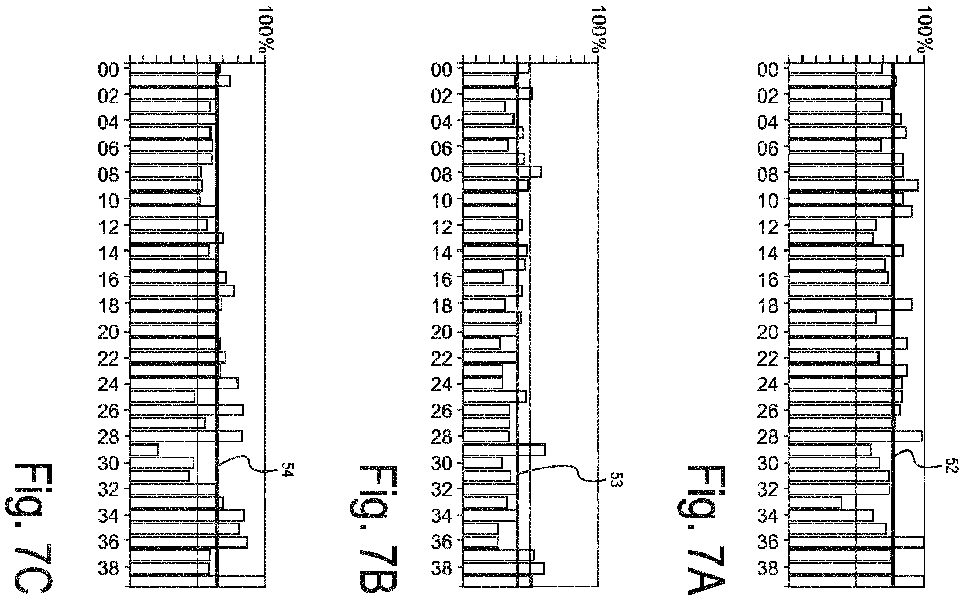

Figs. 7A to 7C are examples of such graphical representations. Each of these graphical representations is generated using quality parameter values from a plurality of images and imaging parameter values, wherein the imaging parameters are device identification numbers, which uniquely identify the imaging units. Each of the graphical representations which are shown in Figs. 7A to 7C, represents an analysis based on images, which have been acquired using 40 imaging units having device identification numbers ranging from 0 to 39.

Fig. 7A is a histogram of the percentage of images which meet the field of view quality requirements (Y-axis) over the device identification number (X-axis). In the example of Fig. 7A, the field of view quality requirements relate to one or more of the quality parameters which have been discussed with reference to Figs. 2A to 2C. The horizontal line 52 in Fig. 7A represents the average of the percentages calculated over all imaging units. As can be seen from Fig. 7A, the imaging unit having the identification number 33 generates a comparatively high percentage of images, which do not meet the field of view quality requirements, whereas the images, which are generated using the imaging units having the numbers 28, 36 and 40, all meet the field of view quality requirements. The performance achieved by the imaging units 26 and 40 even demonstrate that it is possible to achieve a percentage of 100%.

Fig. 7B is a histogram of the percentage of images, which meet the inhalation state quality requirements (Y-axis) versus the identification number of the imaging unit (X-axis). The horizontal line 53 in Fig. 7B represents the average of the percentages calculated over all imaging units. In the example of Fig. 7B, the inhalation state quality requirements relate to one or more of the inhalation state parameters which have been discussed with reference to Figs. 3A and 3B. As can be seen from the histogram of Fig. 7B, for almost all of the imaging devices, the percentage of images which meet the requirements for the inhalation state quality parameters is less than 50%. This indicates that the image quality in the radiology department can be significantly improved by ensuring that the inhalation state quality requirements are met.

Fig. 7C is a histogram of the percentage of images, which meet the rotation angle quality requirements (Y-axis) versus the identification number of the imaging unit (X-axis). In the example of Fig. 7C, the rotation angle quality requirements relate to the rotation angle about the subject's longitudinal axis, which has been discussed with reference to Figs. 4A to 5B. The horizontal line 54 in Fig. 7C represents the average of the percentages calculated over all imaging units. The average value of almost 70%, indicated by the horizontal line 54, is acceptable but should be improved, e.g. by better instructing the radiographers. Further, as is demonstrated by the performance of the imaging unit having the identification number 39, a well-trained radiographer can achieve a percentage of 100%.

Additionally or alternatively, the data processing system is configured to determine, in an automated or semi-automated manner (i.e. requiring user intervention), an analysis parameter and/or a graphical representation depending on one or more quality parameter values and/or a time and date when the image is acquired. This allows the data processing system to analyze (via one or more determined analysis parameter values) and/or visualize (via one or more graphical representations) changes in image quality over time. By way of example, quality parameters are tracked over a predetermined period of time and presented to the user using a graphical representation. The graphical representation may be in the form of a trend line. Thereby, for example, the radiographer can see, whether changes in positioning techniques lead to a reduction of positioning errors.

The above discussion indicates that the automatic determination of the quality parameter values allow tracking down the reasons why an inacceptable number of the images fail to meet the requirements for one or more of the quality parameters. Specifically, an analysis which is based on the quality parameter values and the imaging parameter values allows obtaining a better understanding, which factors can cause an insufficient performance for one or more of the quality parameter values. Based on the determined analysis parameter values, the data processing system may perform a root-cause analysis. The root-cause analysis may link observed quality issues (i.e. observed using the determined quality parameter values) to factors, which cause them.

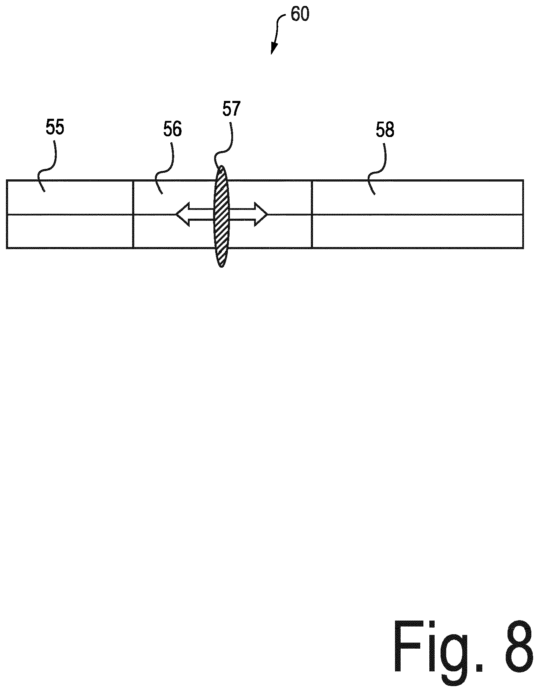

Fig. 8 is a schematic illustration of a user input element 60 of a graphical user interface which is displayed by the computer system 7 (shown in Fig. 1) to the user, which may be a radiologist who reviews the images which have been acquired using the imaging units 2a to 2c. The user input element 60 is configured as a slider control which allows the user to provide input to the computer system 7 by moving a slider 57 using the computer mouse. The input is indicative of a level of acceptance of an image, which is presented to the user via the graphical user interface. The input element 60 is displayed to the user concurrently with an image and is configured to allow the user to choose, based on the displayed image, between three levels of acceptance for this image. A first level of acceptance 55 indicates that the image is unsuitable for diagnosis. A second level of acceptance 56 indicates that the image is acceptable, but an improvement in one or more image quality parameter values is desirable. This level represents an easy to reach threshold, which is surpassed by images which allow a diagnosis but which have a quality level which is less than a pre-defined level.

By way of example, the radiologist may accept under-collimated images since they allow performing a diagnosis. Under-collimated images, however, incur an avoidable radiation exposure to body portions of the subject, which are located outside the lung field. As a further example, if the radiologists accepts an image for a specific diagnosis, this does not mean that the image is well comparable with previously acquired images from the same subject or comparable with images from other subjects. It does also not mean that the image is suitable for other diagnoses.

A third level of acceptance 57 is provided in order to allow the user to indicate that the image is suitable for diagnosis and that it is not necessary to take any measures to improve quality parameter values. The third level of acceptance 57 therefore represents the quality which the radiologist expects to be feasible. The three levels of acceptance allow establishing a high quality level in a radiology department without the need to reject images and to force a retake with all its consequences. By way of example, a strongly under-collimated image could, for instance, reach the lower threshold to avoid a retake for the patient, but would miss the second threshold, thus indicating a quality problem to be addressed on the long run in case it happens too often or very pronounced.

It is conceivable that the user interface is configured to allow the user to select between more than than three levels of acceptance. It is also conceivable, that the input element 60 only provides two levels of acceptance. By way of example, a first level may be provided indicating that the image is suitable for diagnosis and a second level may be provided indicating that the image is unsuitable for diagnosis.

Alternatively, the user interface of the computer system 7 may be configured so that a plurality of inputs can be provided to the computer system 7 independently from each other. A first level of acceptance may be provided for one or more quality parameters which relate to the field of view. Additionally or alternatively, a second level of acceptance may be provided for one or more quality parameters, which relate to the inhalation state. Additionally or alternatively, a third level of acceptance may be provided for one or more quality parameters, which relate to the rotation of the subject relative to the radiation detector. This allows a more accurate analysis based on the user input.

The one or more input elements 60 allow the radiologist to input to the computer system 7 the result of his assessment of the quality of the image. The data processing system then determines one or more analysis parameters and/or one or more graphical representations based on the user input and further based on one or more of the quality parameter values, which have been determined using the data processing system.

Specifically, the user input allows determination of one or more analysis parameters depending on one or more quality parameter values on the one hand and one or more acceptance level settings (received via user input) for one or more quality parameters on the other hand. The analysis parameter may be a parameter of a statistical relationship between one or more of the quality parameter values on the one hand and the acceptance level settings on the other hand.

Thereby, the input which is provided via one or more of the input elements 60 allows a comparison between the automatically or semi-automatically (i.e. requiring user intervention) determined quality parameter values on the one hand and the feedback provided by the radiologist on the other hand. It further allows adaptation of the requirements for one or more of the quality parameters.

Specifically, the data processing system is further configured to use the input provided by the radiologist via one or more of the input elements 60 and one or more quality parameter values to determine quality requirements, such as ranges or thresholds, for one or more of the quality parameters. The quality requirements may be determined depending on one or more of the analysis parameters.

Additionally or alternatively, the input provided by the radiologist via one or more of the input elements 60 may be used to train a machine learning algorithm of the data processing system which is configured to determine, based on the quality parameter values, whether or not the respective image is suitable for diagnosis. Thereby, using the machine learning algorithm, based on the quality parameter values, the image quality can be automatically assessed by the data processing system without using further input from the radiologist.

The quality requirements may relate to a person (e.g. a certain radiologist) who performs the diagnosis based on the image and/or relate to a diagnosis (e.g. to TB diagnosis) which is to be performed based on the images. The quality requirements, which are determined based on the user input can be used in the following two scenarios.

Firstly, in a discussion of the results of the determined quality parameter values and the quality requirements, which are determined depending on the radiologist's input via one or more of the input elements 60, both the radiographer (in the function of a provider) and the radiologist (in the function of a consumer) may question and probably modify their behavior in order to improve their collaboration so that an improved quality level is achieved. Specifically, the data will help to find out, where it is worth spending additional effort for improving image quality and where effort can be spared.

Secondly, if the communication between the radiographer (provider) and the radiologist (consumer) is impeded (e.g. due to long distance, time restrictions, or where at least one of both roles is not properly trained for the task), an automated quality assessment system trained for the requirements of this specific situation is very helpful, because unlike pure standards, they reflect what is required by experts in that role and still achievable under the given circumstances.

Additionally or alternatively, the data processing system may be configured to generate a graphical representation depending on one or more quality parameter values and one or more acceptance level settings received via the user input. The graphical representations may be generated automatically or interactively (i.e. requiring user intervention) and may be displayed to the user using a graphical user interface. The graphical representations help the radiographer and the radiologists in their discussions to identify factors which cause an insufficient image quality performance and to determine which action is needed.

Thereby, a system and a method are provided which allow a more efficient quality control for medical images.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the protective scope of the claims of the present invention. In the claims, the word "comprising" does not exclude other elements or steps, and the indefinite article "a" or "an" does not exclude a plurality. Any reference signs in the claims should not be construed as limiting the scope.

## Claims

1. A system (1) for analysis of medical image data, which represent one or more two-dimensional or three-dimensional medical images, which are acquired from one or more human subjects (8a) using one or more imaging units (2a), wherein each of the imaging units (2a) is configured to acquire images;
wherein the system (1) comprises a data processing system, which is configured to read and/or generate, for each of the medical images, one or more quality parameter values, of a position parameter and/or an orientation parameter of one or more body portions of the subject relative to each other or relative to the respective imaging unit;
wherein the data processing system is configured for automatically or interactively performing at least one of (a) and (b):
(a) reading and/or generating, for each of one or more of the medical images, one or more imaging parameter values, which are indicative of one or more properties of the respective imaging unit (2a), one or more properties of an operator, who operates the respective imaging unit, and/or one or more properties of a subject (8a) from which the image is acquired; and
determining at least one graphical representation which is displayed by the data processing system and/or at least one analysis parameter value, each of which depending on one or more of the quality parameter values and one or more of the imaging parameter values; and/or
(b) receiving and/or reading, for each of one or more of the medical images, user input which is indicative of a level of acceptance of the respective image, and
determining at least one graphical representation which is displayed by the data processing system and/or at least one analysis parameter value, each of which depending on one or more of the quality parameter values and at least a portion of the user input.

2. The system (1) of claim 1, wherein at least (a) applies and the graphical representation and/or the at least one analysis parameter values each of which being determined depending on the one or more of the quality parameter values and the one or more of the imaging parameter values, are indicative of one or more parameters of a relationship between the one or more of the imaging parameter values on the one hand and the one or more of the quality parameter values on the other hand.

3. The system (1) of claim 1 or 2, wherein at least (b) applies and the graphical representation and/or the at least one analysis parameter values, each of which being determined depending on one or more of the quality parameter values and at least a portion of the user input are indicative of one or more parameters of a relationship between the portion of the user input on the one hand and the one or more of the quality parameter values on the other hand.

4. The system (1) of any one of the preceding claims, wherein at least (b) applies and the data processing system is configured to determine the one or more analysis parameter values, each of which depending on one or more of the quality parameter values and at least a portion of the user input;
wherein the data processing system is further configured to determine an acceptance level for a further medical image depending on the determined one or more analysis parameter values.

5. The system (1) of any one of the preceding claims, wherein at least (b) applies and the data processing system is configured to determine the one or more analysis parameter values, each of which depending on one or more of the quality parameter values and at least a portion of the user input;
wherein the determined one or more analysis parameter values are operation parameter values of a machine learning algorithm for automatically determining an acceptance level for a further medical image depending on one or more quality parameter values of the respective image.

6. The system (1) of any one of the preceding claims, wherein at least (b) applies and for each of a plurality of the quality parameters, the user input is indicative of a separate level of acceptance for the respective quality parameter.

7. The system (1) of any one of the preceding claims, wherein the data processing system is configured so that for each of the medical images, the one or more quality parameters comprise one or a combination of (i) to (iii):
(i) one or more parameters of a position of a lung field (10) within a two-dimensional field of view of the imaging unit (2a) and/or one or more parameters of an extent of the lung field (10) relative to the field of view;
(ii) one or more parameters of an orientation of at least one of the body portions relative to the imaging system (2a); and/or
(iii) one or more parameters of an inhalation state of the subject during acquisition of the respective image.

8. The system (1) of any one of the preceding claims, wherein the data processing system is configured to identify one or more of the medical images depending on the quality parameter values.

9. The system (1) of claim 8, wherein the one or more medical images are identified depending on one or more thresholds and/or depending on one or more ranges for the one or more quality parameters.

10. The system (1) of any one of the preceding claims, wherein the data processing system is configured to display for one or more of the medical images, a graphical representation (45, 46), which is indicative of a parameter of a corrective movement of the subject (8a) from which the image has been acquired.

11. The system (1) of claim 10, wherein the graphical representation (46) includes an animation sequence for visualizing the parameter of the corrective movement.

12. The system (1) of any one of the preceding claims, wherein the data processing system is configured to use an image analysis algorithm, which is used for determining one or more of the quality parameter values using at least a portion of the medical image data;
wherein the image analysis algorithm is based on machine learning.

13. A method for analyzing medical image data, wherein the medical image data represent one or more two-dimensional or three-dimensional medical images which are acquired from one or more human subjects (8a) using one or more imaging units (2a), wherein each of the imaging units (2a) is configured to acquire images;
wherein the method comprises:
reading and/or generating, using the data processing system, for each of the medical images, one or more quality parameter values, of a position parameter and/or an orientation parameter of one or more body portions of the subject (8a) relative to each other or relative to the respective imaging unit;
wherein the method further comprises one or a combination of (a) and (b):
(a) reading and/or generating, using the data processing system, for each of one or more of the medical images, one or more imaging parameter values, which are indicative of one or more properties of the respective imaging unit (2a), one or more properties of an operator, who operates the respective imaging unit (2a), and/or one or more properties of a subject (8a) from which the image is acquired; and
determining, using the data processing system, at least one graphical representation which is displayed by the data processing system and/or at least one analysis parameter value, each of which depending on one or more of the quality parameter values and one or more of the imaging parameter values; and/or
(b) receiving and/or reading, using the data processing system, for each of one or more of the medical images, user input which is indicative of a level of acceptance of the respective image, and
determining, using the data processing system, at least one graphical representation which is displayed by the data processing system and/or at least one analysis parameter value, each of which depending on one or more of the quality parameter values and at least a portion of the user input.

14. A program element for analyzing medical image data, wherein the medical image data represent one or more two-dimensional or three-dimensional medical images which are acquired from one or more human subjects (8a) using one or more imaging units (2a), wherein each of the imaging units (2a) is configured to acquire images;
wherein the program element, when being executed by a processor of the data processing system, is adapted to carry out:
reading and/or generating, using the data processing system, for each of the medical images, one or more quality parameter values of a position parameter and/or an orientation parameter of one or more body portions of the subject (8a) relative to each other or relative to the respective imaging unit (2a);
wherein the program element, when being executed by a processor of the data processing system, is adapted to carry out one or a combination of (a) and (b):
(a) reading and/or generating, using the data processing system, for each of one or more of the medical images, one or more imaging parameter values, which are indicative of one or more properties of the imaging unit (2a), one or more properties of an operator, who operates the respective imaging unit (2a), and/or one or more properties of a subject (8a) from which the image is acquired; and
determining, using the data processing system, at least one graphical representation which is displayed by the data processing system and/or at least one analysis parameter value, each of which depending on one or more of the quality parameter values and one or more of the imaging parameter values; and/or
(b) receiving and/or reading, using the data processing system, for each of one or more of the images, user input which is indicative of a level of acceptance of the respective image, and
determining, using the data processing system, at least one graphical representation which is displayed by the data processing system and/or at least one analysis parameter value, each of which depending on one or more of the quality parameter values and at least a portion of the user input.

15. A computer readable medium having stored thereon the computer program element of claim 14.
